# EUROPEAN PATENT SPECIFICATION

(11) **EP 2 231 104 B1**
(45) Date of publication and mention of the grant of the patent: **18.12.2013**
(21) Application number: 08868881.7
(22) Date of filing: 22.12.2008
(51) Int. Cl.: A61K 8/04, A61K 8/31, A61K 8/36, A61K 8/37, A61K 8/39, A61Q 9/02, A61Q 19/10, A61Q 19/00, A61K 8/34, A61K 8/73

(54) **PERSONAL CARE COMPOSITIONS CONTAINING SKIN CONDITIONING AGENTS**
KÖRPERPFLEGEZUSAMMENSETZUNGEN, DIE SUBSTANZEN ZUR VERBESSERUNG DES HAUTZUSTANDS ENTHALTEN
COMPOSITIONS D'HYGIÈNE CORPORELLE CONTENANT DES AGENTS DE CONDITIONNEMENT DE LA PEAU

(30) Priority: 26.12.2007 US 9152
(43) Date of publication of application: 29.09.2010
(73) Proprietor: The Procter & Gamble Company, Cincinnati, OH 45202 (US)
(72) Inventor: KYTE, Kenneth, Eugene, III, Lebanon Ohio 45036 (US)
(74) Representative: Kohol, Sonia
(86) International application number: PCT/IB2008/055492
(87) International publication number: WO 2009/083895

(56) References cited:
- WO-A-90/11343
- WO-A-94/02109
- WO-A-03/028676
- WO-A-2004/009047
- FR-A- 2 731 616
- US-A- 3 541 581
- US-A- 4 708 813
- US-A- 6 165 456

## Description

### FIELD OF THE INVENTION

The present invention is directed to personal care compositions containing skin conditioning agents. The personal care compositions may be used or marketed for a variety of applications including, for example, cleansing, shaving, and/or moisturizing one's skin.

### BACKGROUND OF THE INVENTION

Conventional skin cleansing liquid products, shower gels, and shave gels are usually thick liquids that are relatively slow foaming and produce very little, relatively weak foam that quickly flattens. Post-foaming gels such as shaving gels use a soap-based system that can optionally contain a minor amount of surfactant. Soap-based shaving gels can be extremely sensitive to even small additions of commonly used personal care benefit agents, such as, for example, lipids, emollients, and vitamins. The addition of these benefit agents can cause a drastic loss of structure in the overall composition such that the end product more closely resembles a lotion than a shaving gel. WO2004/009047, US3541581, WO03/028676, WO94/02109, US6165456, WO90/11343, US4708813 and FR2731616 include exemplary personal care compositions.

Known post-foaming shower gels consist of conventionally thickened sodium lauryl ether sulfate (SLES) and fatty acid diethanolamide (CDEA) blended together with a low level of a foaming agent (e.g., liquefied hydrocarbon and chlorofluorohydrocarbon propellants). These shower gels can suffer from a variety of shortcomings. For example, the post-foaming shower gels can exhibit a stringy, tacky feel; they can possess poor high temperature stability; and/or they can be restricted to low levels of foaming agent due to a thinning effect of foaming agents on the SLES/CDEA base, which can result in non-optimization of foaming properties. These post-foaming shower gels typically require a high viscosity base to allow for any thinning effect from the foaming agent while still retaining desired gel properties; however, the high viscosity aesthetic property can present process difficulties.

Accordingly, there is a need for post-foaming personal care products that are capable of employing relatively high levels of skin conditioning agents (and/or other skin benefit agents) and still maintain gel structure and application feel properties.

### DETAILED DESCRIPTION OF THE INVENTION

The present invention may be understood more readily by reference to the following detailed description of illustrative and preferred embodiments. It is to be understood that the

scope of the claims is not limited to the specific ingredients, methods, conditions, devices, or parameters described herein, and that the terminology used herein is not intended to be limiting of the claimed invention. Also, as used in the specification, including the appended claims, the singular forms "a," "an," and "the" include the plural, and reference to a particular numerical value includes at least that particular value, unless the context clearly dictates otherwise. When a range of values is expressed, another embodiment includes from the one particular value and/or to the other particular value. Similarly, when values are expressed as approximations, by use of the antecedent basis "about," it will be understood that the particular values form another embodiment. All ranges are inclusive and combinable.

The compositions/methods of the present invention can comprise, consist of, and consist essentially of the features and/or steps of the invention described herein, as well as any of the additional or optional ingredients, components, steps, or limitations described herein.

A variety of methods are including in the description and appended claims, which include a listing of steps with either letter or numerical designations associated with the individual steps. It is to be understood that although they may, the methods and steps do not necessarily need to be performed in the order of listing, or in accordance with their associated designations; for example, a step (c) may be performed before or after a step (b). Furthermore, although steps are listed individually, some steps may be performed simultaneously with other steps. Alternatively, the steps are all performed sequentially. Timing of the steps can vary. Also, there may or may not be delays between steps. And the methods described herein may include other steps than those explicitly listed and/or recited in the appended claims.

### I. Personal Care Compositions

The present invention is directed to personal care compositions that include an aqueous base composition and a volatile post foaming agent mixed with the base composition. The base composition comprises a lipophilic skin conditioning agent, a structuring agent, and a water dispersible surface active agent wherein the structuring agent is selected from the group consisting of isododecane, isohexadecane, isoeicosane, glyceryl oleate, polyglyceryl diisostearate, and mixtures thereof, and wherein the water dispersible surface active agent is selected from the group consisting of a soap, interrupted soap and mixtures thereof. These components and additional optional components will be discussed more fully below. Exemplary base compositions include from about 50% to about 80% water.

### A. Lipophilic Skin Conditioning Agent

Personal care compositions of the present invention employ one or more lipophilic skin conditioning agents. The concentration level of the skin conditioning agents either singularly or collectively may range from about 1% to about 12% by weight of the base composition. Some preferred concentration levels include greater than about 3%, from about 3% to about 10%, and from about 5% to about 8%. It is to be understood that the scope of appended claims that do not specify a concentration level of the lipophilic skin conditioning agent is not limited to the levels described in this paragraph.

Exemplary skin conditioning agents include a hydrocarbon or polymeric hydrocarbon selected from the group consisting of mineral oil, isoparaffin, greater than C20 hydrogenated polyisobutene, and petrolatum; and an ester composed of a branched C16-C22 alkyl chain and a mono alkyl group consisting of a linear or branched C1 to C6 alkyl chain. One preferred skin conditioning agent comprises an isostearic acid derivative; for example, isostearyl isostearate, isopropyl isostearate, and mixtures thereof. Other skin conditioning agents known to the skilled artisan may also be employed depending on the form of the personal care composition and the targeted skin benefit.

The skin conditioning agents may also help to reduce the coefficient of friction for personal care compositions provided herein that are in the form of shaving compositions. The reduction in friction can decrease the potential for skin irritation that can arise from contacting the skin one or more times with a razor blade. Employment of the skin conditioning agent in this context may also permit formulation flexibility regarding the type and concentration level of lubricants (as discussed more fully below) that are included in the shaving compositions.

### B. Structuring Agent

Personal care compositions of the present invention employ one or more structuring agents. The concentration level of the structuring agents either singularly or collectively may range from about 1% to about 10% by weight of the base composition. Some preferred concentration levels include from about 1% to about 5%, and from about 3% to about 7. It is to be understood that the scope of appended claims that do not specify a concentration level of the structuring agent is not limited to the levels described in this paragraph.

Exemplary structuring agents comprise a branched or unsaturated hydrocarbon moiety, including, for example, C8 to C20 branched alkanes, glyceryl or polyglyceryl mono or diesters of branched or unsaturated fatty acids, and mixtures thereof. In the present invention, the structuring agents are isododecane, isohexadecane, isoeicosane, glyceryl oleate, polyglyceryl diisostearate, and mixtures thereof. Other structuring agents known to the skilled artisan may also be employed depending on the form of the personal care composition and the targeted skin benefit.

### C. Water Dispersible Surface Active Agent

Personal care compositions of the present invention contain one or more water dispersible surface active agents. The water dispersible surface active agent is preferably one that is capable of forming lather and comprises a soap, an interrupted soap, or a mixture of one or more of these. Exemplary embodiments employ one or more surface active agents at total levels of from about 5% to about 15% or 20%, by weight of the base composition. In some preferred embodiments, surface active agents are employed at less than 20% by weight of the base composition to reduce the potential for over drying/irritation and/or counter balancing benefits that may be derived from incorporation of the lipophilic skin conditioning agent. Other concentration levels of the water dispersible surface active agent may optionally be employed and still fit within the spirit of the present invention.

Soaps may include, for example, the sodium, potassium and lower alkanolamine (preferably triethanolamine) salts of C12 22, preferably C14 18, fatty acids. Typical fatty acids include lauric, myristic, palmitic and stearic acid and mixtures thereof. The preferred fatty acids are palmitic and stearic. The interrupted soaps may include, for example, the sodium, potassium and lower alkanolamine (preferably triethanolamine) salts of N-fatty acyl sarcosines, wherein the fatty acyl moiety has 12 to 22, preferably 14 to 18, carbon atoms. Typical sarcosines include stearoyl sarcosine, myristoyl sarcosine, palmitoyl sarcosine, oleoyl sarcosine, lauroyl sarcosine, cocoyl sarcosine and mixtures thereof. The soaps and the interrupted soaps may be utilized in the preneutralized form (i.e., as the sodium, potassium or alkanolamine salt) or in the free acid form followed by subsequent neutralization with sodium hydroxide, potassium hydroxide and/or lower alkanolamine (preferably triethanolamine). In any event, the final shaving composition preferably contains sufficient base to neutralize or partially neutralize the soap component and adjust the pH to the desired level (typically between 5 and 10, more typically between 6 and 9). It is most preferred that the shaving composition includes a soap (e.g., triethanolamine palmitate/stearate), an interrupted soap (e.g., triethanolamine stearoyl/myristoyl sarcosinate), or a mixture thereof.

The water dispersible surface active agent may also optionally include a non-ionic, amphoteric and/or anionic surfactant. Suitable non-ionic surfactants will typically have an HLB of 9 or more and include the polyoxyethylene ethers of fatty alcohols, acids and amides, particularly those having 10 to 20, preferably 12 to 18, carbon atoms in the fatty moiety and about 2 to 60, preferably 4 to 30, ethylene oxide units. These include, for example, Oleth 20, Steareth 21, Ceteth 20, Laureth 4 and Laureth 23. Other non-ionic surfactants include the polyoxyethylene ethers of alkyl substituted phenols, such as Nonoxynol-4 and Nonoxynol-20, fatty alkanolamides such as Lauramide DEA and Cocamide MEA, polyethoxylated sorbitan esters of fatty acids, such as Polysorbate 20, lauryl polyglucoside, sucrose laurate, and polyglycerol 8 oleate. Suitable amphoteric surfactants include, for example, the betaines and sultaines such as cocoamidopropyl betaine, coco dimethyl carboxymethyl betaine, coco sultaine and the like. Suitable anionic surfactants include, for example, the sodium, potassium, ammonium and substituted ammonium salts (such as the mono-, di- and triethanolamine salts) of C8 C22, preferably C12 C18, alkyl sulfates (e.g., sodium lauryl sulfate, ammonium lauryl sulfate), alkyl sulfonates (e.g., ammonium lauryl sulfonate), alkylbenzene sulfonates (e.g., ammonium xylene sulfonate), acyl isethionates (e.g., sodium cocoyl isethionate), acyl lactylates (e.g., sodium cocoyl lactylate) and alkyl ether sulfates (e.g., ammonium laureth sulfate). The surface active agent may typically include up to about 10%, preferably 1 to 8%, of non-ionic, amphoteric and/or anionic surfactants.

### D. Optional Components

Personal care compositions of the present invention may employ one or more lubricants, particularly when the compositions are in the form of a shaving composition. Exemplary lubricants include lubricous water soluble polymers, water insoluble particles, and hydrogel-forming (or water swellable) polymers. Species of these exemplary lubricants are discussed below.

Useful lubricious water soluble polymers may have a molecular weight greater between about 300,000 and 15,000,000 daltons, preferably more than about one million daltons, and will include a sufficient number of hydrophilic moieties or substituents on the polymer chain to render the polymer water soluble. The polymer may be a homopolymer, copolymer or terpolymer. Examples of suitable lubricious water soluble polymers include polyethylene oxide, polyvinylpyrrolidone, and polyacrylamide. A preferred lubricious water soluble polymer comprises polyethylene oxide, and more particularly a polyethylene oxide with a molecular weight of about 1 to about 5 million daltons. Particularly suitable polyethylene oxides include, for example, PEG 23M (MW ≈ 1 million), PEG 45M (MW ≈ 2 million) and PEG 90M (MW ≈ 4 million).

Useful water insoluble particles may include inorganic particles or organic polymer particles. Examples of inorganic particles include titanium dioxide, silicas, silicates and glass beads. Examples of organic polymer particles include polytetrafluoroethylene particles, polyethylene particles, polypropylene particles, polyurethane particles, polyamide particles, or mixtures of two or more of such particles. Any of the forgoing particles may also include a surface treatment to make the particles more readily dispersible or improve their cosmetic aesthetics. Preferred are polytetrafluoroethylene particles (e.g., PTFE particles available from MicroPowders, Inc. under the tradename Microslip). Preferably, the water insoluble particles will have an average particle size of about 1 µm to about 100 µm, more preferably about 2 µm to about 50 µm, and most preferably about 5 µm to about 15µm. The particles may be of any desired shape including spherical bead, elongated fiber or irregular shape, with spherical bead being the preferred shape.

Hydrogel-forming polymers are typically highly hydrophilic polymers that, in water, form organized three-dimensional domains of approximately nanometer scale. The hydrogel-forming polymer generally has a molecular weight greater than about one million daltons (although lower molecular weights are possible) and typically is at least partially or lightly crosslinked and may be at least partially water insoluble, but it also includes a sufficient number of hydrophilic moieties so as to enable the polymer to trap or bind a substantial amount of water within the polymer matrix and thereby form three-dimensional domains. It has been found that personal care compositions (e.g., shave gels) that include the hydrogel-forming polymer have improved gel structure and reduced coefficient of friction (i.e., increased lubricity). Examples of suitable hydrogel-forming polymers include a polyacrylic acid or polymethacrylic acid partially esterified with a polyhydric alcohol; hydrophilic polyurethanes; lightly crosslinked polyethylene oxide; lightly crosslinked polyvinyl alcohol; lightly crosslinked polyacrylamide; hydrophobically modified hydroxyalkyl cellulose; hydroxyethyl methacrylate; and crosslinked hyaluronic acid.

One preferred hydrogel-forming polymer comprises polyacrylic acid partially esterified (e.g., about 40% to 60%, preferably about 50%, esterified) with glycerin. Such a polymer includes glyceryl acrylate/acrylic acid copolymer (MW > one million). It is believed that the glyceryl acrylate/acrylic acid copolymer forms a clathrate that holds water, which, upon release supplies lubrication and moisturization to the skin. A preferred source of glyceryl acrylate/acrylic acid copolymer is available from ISP Technologies, Inc. (United Guardian Inc.) under the tradename Lubrajel®, particular the form known as Lubrajel® oil which contains about 1.0%-1.3% glyceryl acrylate/acrylic acid copolymer in aqueous glycerin (~ 40% glycerin). Lubrajel® oil also includes about 0.6% PVM/MA copolymer (also known as methoxyethylene/maleic anhydride copolymer), which may further contribute to the lubricity of this source.

Additional structuring agents or rheology modifiers may be employed beyond that of the structuring agent component discussed above. These additional structuring agents may include, for example, hydroxyalkyl cellulose polymers such as hydroxyethyl cellulose and hydroxypropyl cellulose (sold under the trademarks "Natrosol" and "Klucel" respectively), PEG-150 distearate, carboxymethyl cellulose, and cellulose methyl ether (sold under the trademark "Methocel"). Other suitable structuring agents may include polysaccharide gums such as, for example, xanthan gum, carrageenan gum, guar gum, locust bean gum, and hydroxypropyl guar gum.

Although not necessary to forming a useful personal care composition, other cosmetic ingredients may be advantageously added to improve the application aesthetics and/or achieve other benefits. For example, the composition may include one or more of the following components: beard wetting agents, additional skin conditioning agents to those described above (e.g., vitamins A, C and E, aloe, allantoin, panthenol, alpha-hydroxy acids, phospholipids, triglycerides, botanical oils, amino acids), foam boosters, emollients, humectants (e.g., glycerin, sorbitol, propylene glycol), fragrances, colorants, antioxidants, preservatives, etc.

### E. Volatile Post Foaming Agent

A volatile post foaming agent is mixed with the base composition to enable the final personal care composition to foam when dispensed from an appropriate container. Exemplary post foaming agents include saturated aliphatic hydrocarbons having 4 to 6 carbon atoms, such as n pentane, isopentane, neopentane, n butane, isobutane, and mixtures thereof. The post-foaming agent may be selected so as to provide a vapor pressure at 20°C of about 3 to about 20 psig, preferably about 5 to about 15 psig. Other volatile post foaming agents known to the skilled artisan may also be employed.

### II. Methods

The present invention also encompasses a variety of methods. A method of shaving is provided, including the steps of: applying a volume of a personal care composition as described herein to an area of skin to be shaved, shaving the area of skin to be shaved, and rinsing the area of skin with water. It is desirable that a portion of the lipophilic skin conditioning agent remain on the surface of the skin after it is shaved and rinsed. The concentration level of the lipophilic skin conditioning agent and instructions for using the personal care composition may be adjusted depending on the amount of skin conditioning agent is desired to remain after a shaving experience.

A method of merchandising a personal care composition is provided, including the steps of: offering for sale a personal care composition as described herein, and communicating to consumers the personal care composition is a shaving composition that is targeted for sensitive skin, dry skin, or that it is marketed as a moisturizing composition.

A method of structuring a shave gel composition that includes a lipophilic skin conditioning agent is provided, including the step of employing a structuring agent comprising a branched or unsaturated hydrocarbon moiety. Exemplary lipophilic skin conditioning agents and structuring agents are listed above.

### III. Examples

The following shave gel examples further describe and demonstrate embodiments within the scope of the present invention. The examples are given solely for the purpose of illustration and are not to be construed to limit scope of the present invention as many variations thereof are possible without departing from the spirit and scope of the invention.

| | **Example 1** | **Example 2** | **Example 3** | **Example 4** | **Example 5** |
|---|---|---|---|---|---|
| **Ingredient** | Base 1 | Base 2 | Base 3 | Base 4 | Base 5 |
| Water | QS to 100 | QS to 100 | QS to 100 | QS to 100 | QS to 100 |
| Palmitic Acid | 7.75 | 7.75 | 6.20 | 6.20 | 6.20 |
| Triethanolamine | 6.05 | 6.05 | 4.84 | 4.840 | 4.84 |
| Glyceryl Oleate | 3.0 | 3.0 | 3.0 | 3.0 | 3.0 |
| Isohexadecane | 3.0 | 5.0 | 3.0 | 3.0 | 0.0 |
| Stearic Acid | 2.60 | 2.60 | 2.08 | 2.080 | 2.08 |
| Sorbitol | 0.70 | 0.70 | 0.70 | 0.70 | 0.70 |
| Fragrance | 0.50 | 0.75 | 0.65 | 0.850 | 0.85 |
| Hydroxyethylcellulose | 0.50 | 0.50 | 0.50 | 0.50 | 0.50 |
| PEG-90M | 0.06 | 0.075 | 0.075 | 0.09 | 0.09 |
| Isopropyl Isostearate | - | - | 2.0 | 4.50 | 8.0 |
| Petrolatum | 2.0 | 5.0 | - | - | - |
| | | | | | |
| Volatile Post Foaming Agent | 1-4% | 1-4% | 1-4% | 1-4% | 1-4% |
| Bases 1 through 5 | 96-99% | 96-99% | 96-99% | 96-99% | 96-99% |

The shave gel examples can be made as follows: add the water soluble components to water and then mix until the polymers are dissolved (approximately 30 minutes). Heat the mixture and then add the following components at a temperature of about 60°C while continuing to mix: the palmitic/stearic acid, the lipophilic skin conditioning agents, and the structuring agents. Heat the mixture to about 80-85°C prior to adding the triethanolamine, and then mix for about 40 minutes. Cool the mixture down to about 45°C and then add the remaining components while continuing to mix to form the base composition. Add the volatile self-foaming agent (at a temperature of about 5°C) to the base composition (cooled to about 20°C) in a pressurized vessel. Fill bottom-gassed cans with the final composition.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

All documents cited in the Detailed Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A personal care composition, comprising:
(a) an aqueous base composition, comprising:
i) a lipophilic skin conditioning agent;
ii) a structuring agent; and
iii) a water dispersible surface active agent; and
(b) a volatile post-foaming agent mixed with the base composition to form the personal care composition;
wherein the structuring agent is selected from the group consisting of isododecane, isohexadecane, isoeicosane, glyceryl oleate, polyglyceryl diisostearate, and mixtures thereof; and
wherein the water dispersible surface active agent is selected from the group consisting of a soap, an interrupted soap, and mixtures thereof.

2. The personal care composition of claim 1, wherein the lipophilic skin conditioning agent comprises:
(a) a hydrocarbon or polymeric hydrocarbon selected from the group consisting of mineral oil, isoparaffin, greater than C20 hydrogenated polyisobutene, and petrolatum; and/or
(b) an ester composed of a branched C16-C22 alkyl chain and a mono alkyl group consisting of a linear or branched C1 to C6 alkyl chain.

3. The personal care composition of claims 1 and 2, wherein the lipophilic skin conditioning agent comprises an isostearic acid derivative.

4. The personal care composition of claim 3, wherein the isostearic acid derivative is selected from the group consisting of isostearyl isostearate, isopropyl isostearate, and mixtures thereof

5. The personal care composition of any preceding claim, wherein the lipophilic skin conditioning agent comprises an isostearic acid derivative and the structuring agent is selected from the group consisting of isododecane, isohexadecane, isoeicosane, and mixtures thereof

6. The personal care composition of any preceding claim, wherein the lipophilic skin conditioning agent comprises an isopropyl isostearate and the structuring agent comprises isohexadecane.

7. The personal care composition of any preceding claim, wherein the base composition comprises from 50% to 80%, alternatively from 3% to 10%, alternatively 5% to 8% by weight of the base composition, of water.

8. The personal care composition of any preceding claim, wherein the structuring agent concentration is from 1% to 10%, alternatively from 3% to 7% by weight of the base composition.

9. The personal care composition of any preceding claim, wherein the water soluble or water dispersible surface active agent concentration is from 5% to 20%, alternatively from 5% to 15% by weight of the base composition.

10. The personal care composition of any preceding claim, wherein the base composition further comprises one or more lubricants.

11. The personal care composition of any preceding claim, comprising:
(a) an aqueous base composition, comprising:
i) from 1% to 12%, by weight of the base composition, of a lipophilic skin conditioning agent selected from the group consisting of petrolatum, isostearyl isostearate, isopropyl isostearate, glycerol monoisostearate, and mixtures thereof;
ii) from 1% to 5%, by weight of the base composition, of a structuring agent selected from the group consisting of isododecane, isohexadecane, isoeicosane, glyceryl oleate, polyglyceryl diisostearate, and mixtures thereof; and
iii) from 5% to 20%, by weight of the base composition, of a water dispersible surface active agent selected from the group consisting of a soap, an interrupted soap, and mixtures thereof; and
(b) a volatile post-foaming agent mixed with the base composition to form the shaving composition.

## Patentansprüche

1. Körperpflegezusammensetzung, umfassend:
(a) eine wässrige Basiszusammensetzung, umfassend:
I) ein lipophiles Hautkonditioniermittel,
II) ein strukturierendes Mittel,und
III) ein wasserdispergierbares Tensid und
(b) ein flüchtiges Nachschäumungsmittel, das mit der Basiszusammensetzung gemischt ist, um die Körperpflegezusammensetzung zu bilden,
wobei das strukturierende Mittel ausgewählt ist aus der Gruppe bestehend aus Isododecan, Isohexadecan, Isoeicosan, Glyceryloleat, Polyglyceryldiisostearat und Mischungen davon, und
wobei das wasserdispergierbare Tensid ausgewählt ist aus der Gruppe bestehend aus einer Seife, einer unterbrochenen Seife und Mischungen davon.

2. Körperpflegezusammensetzung nach Anspruch 1, wobei das lipophile Hautkonditioniermittel Folgendes umfasst:
(a) einen Kohlenwasserstoff oder polymeren Kohlenwasserstoff, der ausgewählt ist aus der Gruppe bestehend aus Mineralöl, Isoparaffin, hydriertem Polyisobuten mit mehr als C20 und Petrolatum und/oder
(b) einen Ester, der aus einer verzweigten C16-C22-Alkylkette und einer Monoalkylgruppe zusammengesetzt ist, die aus einer linearen oder verzweigten C1-C6-Alkylkette besteht.

3. Körperpflegezusammensetzung nach Anspruch 1 und 2, wobei das lipophile Hautkonditioniermittel ein Isostearinsäurederivat umfasst.

4. Körperpflegezusammensetzung nach Anspruch 3, wobei das Isostearinsäurederivat ausgewählt ist aus der Gruppe bestehend aus Isostearylisostearat, Isopropylisostearat und Mischungen davon.

5. Körperpflegezusammensetzung nach einem der vorstehenden Ansprüche, wobei das lipophile Hautkonditioniermittel ein Isostearinsäurederivat umfasst und das strukturierende Mittel ausgewählt ist aus der Gruppe bestehend aus Isododecan, Isohexadecan, Isoeicosan und Mischungen davon.

6. Körperpflegezusammensetzung nach einem der vorstehenden Ansprüche, wobei das lipophile Hautkonditioniermittel ein Isopropylisostearat umfasst und das strukturierende Mittel Isohexadecan umfasst.

7. Körperpflegezusammensetzung nach einem der vorstehenden Ansprüche, wobei die Basiszusammensetzung zu 50 Gew.-% bis 80 Gew.-%, als Alternative zu 3 Gew.-% bis 10 Gew.-%, als Alternative zu 5 Gew.-% bis 8 Gew.-% der Basiszusammensetzung Wasser umfasst.

8. Körperpflegezusammensetzung nach einem der vorstehenden Ansprüche, wobei die Konzentration des strukturierenden Mittels 1 Gew.-% bis 10 Gew.-%, als Alternative 3 Gew.-% bis 7 Gew.-% der Basiszusammensetzung beträgt.

9. Körperpflegezusammensetzung nach einem der vorstehenden Ansprüche, wobei die Konzentration des wasserlöslichen oder wasserdispergierbaren Tensids 5 Gew.-% bis 20 Gew.-%, als Alternative 5 Gew.-% bis 15 Gew.-% der Basiszusammensetzung beträgt.

10. Körperpflegezusammensetzung nach einem der vorstehenden Ansprüche, wobei die Basiszusammensetzung ferner ein oder mehrere Schmiermittel umfasst.

11. Körperpflegezusammensetzung nach einem der vorstehenden Ansprüche, umfassend:
(a) eine wässrige Basiszusammensetzung, umfassend:
I) zu 1 Gew.-% bis 12 Gew.-% der Basiszusammensetzung ein lipophiles Hautkonditioniermittel, das ausgewählt ist aus der Gruppe bestehend aus Petrolatum, Isostearylisostearat, Isopropylisostearat, Glycerolmonoisostearat und Mischungen davon,
II) zu 1 Gew.-% bis 5 Gew.-% der Basiszusammensetzung ein strukturierendes Mittel, das ausgewählt ist aus der Gruppe bestehend aus Isododecan, Isohexadecan, Isoeicosan, Glyceryloleat, Polyglyceryldiisostearat und Mischungen davon, und
III) zu 5 Gew.-% bis 20 Gew.-% der Basiszusammensetzung ein wasserdispergierbares Tensid, das ausgewählt ist aus der Gruppe bestehend aus einer Seife, einer unterbrochenen Seife und Mischungen davon, und
(b) ein flüchtiges Nachschäumungsmittel, das mit der Basiszusammensetzung gemischt ist, um die Rasierzusammensetzung zu bilden.

## Revendications

1. Composition de soins personnels, comprenant :
(a) une composition de base aqueuse, comprenant :
i) un agent revitalisant pour la peau lipophile ;
ii) un agent structurant ; et
iii)un agent de surface hydrodispersable ; et
(b) un agent post-moussant volatil mélangé avec la composition de base pour former la composition de soins personnels.
dans laquelle l'agent structurant est choisi dans le groupe constitué par l'isododécane, l'isohexadécane, l'isoeicosane, l'oléate de glycéryle, le diisostéarate de polyglycéryle, et leurs mélanges; et
dans laquelle l'agent de surface hydrodispersable est choisi dans le groupe constitué par un savon, un savon interrompu, et leurs mélanges.

2. Composition de soins personnels selon la revendication 1, dans laquelle l'agent revitalisant pour la peau lipophile comprend :
(a) un hydrocarbure ou un hydrocarbure polymère choisi dans le groupe constitué par l'huile minérale, l'isoparaffine, le polyisobutène hydrogéné supérieur à C20, et la vaseline ; et/ou
(b) un ester composé d'une chaîne alkyle en C16 à C22 ramifiée et d'un groupe mono-alkyle constitué d'une chaîne alkyle en C1 à C6 linéaire ou ramifiée.

3. Composition de soins personnels selon les revendications 1 et 2, dans laquelle l'agent revitalisant pour la peau lipophile comprend un dérivé d'acide isostéarique.

4. Composition de soins personnels selon la revendication 3, dans laquelle le dérivé d'acide isostéarique est choisi dans le groupe constitué par l'isostéarate d'isostéaryle, l'isostéarate d'isopropyle, et leurs mélanges.

5. Composition de soins personnels selon l'une quelconque des revendications précédentes, dans laquelle l'agent revitalisant pour la peau lipophile comprend un dérivé d'acide isostéarique et l'agent structurant est choisi dans le groupe constitué par l'isododécane, l'isohexadécane, l'isoeicosane, et leurs mélanges.

6. Composition de soins personnels selon l'une quelconque des revendications précédentes, dans laquelle l'agent revitalisant pour la peau lipophile comprend un isostéarate d'isopropyle et l'agent structurant comprend de l'isohexadécane.

7. Composition de soins personnels selon l'une quelconque des revendications précédentes, dans laquelle la composition de base comprend de 50 % à 80 %, en variante de 3 % à 10 %, en variante de 5 % à 8 % en poids de la composition de base, d'eau.

8. Composition de soins personnels selon l'une quelconque des revendications précédentes, dans laquelle la concentration en agent structurant est de 1 % à 10 %, en variante, de 3 % à 7 % en poids de la composition de base.

9. Composition de soins personnels selon l'une quelconque des revendications précédentes, dans laquelle la concentration de l'agent de surface hydrosoluble ou hydrodispersable est de 5 % à 20 %, en variante de 5 % à 15 % en poids de la composition de base.

10. Composition de soins personnels selon l'une quelconque des revendications précédentes, dans laquelle la composition de base comprend en outre un ou plusieurs lubrifiants.

11. Composition de soins personnels selon l'une quelconque des revendications précédentes, comprenant :
(a) une composition de base aqueuse, comprenant :
i) de 1 % à 12 % en poids de la composition de base, d'un agent revitalisant pour la peau lipophile choisi dans le groupe constitué par la vaseline, l'isostéarate d'isostéaryle, l'isostéarate d'isopropyle, le monoisostéarate de glycérol, et leurs mélanges ;
ii) de 1 % à 5 % en poids de la composition de base, d'un agent structurant choisi dans le groupe constitué par l'isododécane, l'isohexadécane, l'isoeicosane, l'oléate de glycéryle, le diisostéarate de polyglycéryle, et leurs mélanges ; et
iii) de 5 % à 20 % en poids de la composition de base, d'un agent de surface hydrodispersable choisi dans le groupe constitué par un savon, un savon interrompu, et leurs mélanges ; et
(b) un agent post-moussant volatil mélangé avec la composition de base pour former la composition de rasage.
